(19) Europäisches Patentamt
European Patent Office
Office européen des brevets

(11) Veröffentlichungsnummer: **0 137 419**
**B1**

(12) # EUROPÄISCHE PATENTSCHRIFT

(45) Veröffentlichungstag der Patentschrift: **12.08.87**

(51) Int. Cl.⁴: **A 61 K 7/46,** C 07 C 43/23, C 07 C 69/03, C 11 B 9/00

(21) Anmeldenummer: **84111513.2**

(22) Anmeldetag: **27.09.84**

(54) **Riechstoffkompositionen mit einem Gehalt an Benzyläthern.**

(30) Priorität: **07.10.83 CH 5460/83**
**12.07.84 CH 3387/84**

(43) Veröffentlichungstag der Anmeldung: **17.04.85 Patentblatt 85/16**

(45) Bekanntmachung des Hinweises auf die Patenterteilung: **12.08.87 Patentblatt 87/33**

(84) Benannte Vertragsstaaten: **CH DE FR GB LI NL**

(56) Entgegenhaltungen:
**CHEMICAL ABSTRACTS, Band 97, Nr. 10, 6. September 1982, Seite 405, Nr. 78710d, Columbus, Ohio, US**
**PATENTS ABSTRACTS OF JAPAN, Band 7, Nr. 34 (C-150) 1179 , 10. Februar 1983**

(73) Patentinhaber: **L. GIVAUDAN & CIE Société Anonyme, CH- 1214 Vernier- Genève (CH)**

(72) Erfinder: **Ochsner, Paul Albert, Dr., 30 Avenue des Tilleuls, CH- 1203 Genève (CH)**

(74) Vertreter: **Urech, Peter, Dr., Grenzacherstrasse 124 Postfach 3255, CH- 4002 Basel (CH)**

**EP 0 137 419 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

1 Die Erfindung betrifft Riechstoffkompositionen, die durch einen Gehalt an Benzyläthern der Formel

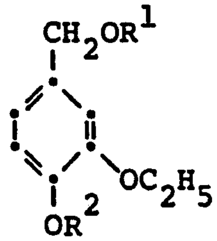

I

worin $R^1$ $C_{1-3}$ Alkyl und $R^2$ Wasserstoff oder $C_{1-4}$-Alkanoyl bedeutet, gekennzeichnet sind.

Die Verbindungen der allgemeinen Formel I weisen besondere organoleptische Eigenschaften auf, auf Grund derer sie sich vorzüglich als Riechstoffe eignen.

Die Verbindungen der allgemeinen Formel I zeichnen sich insbesondere durch eine Kombination von würzigen, rauchig-phenolischen, vanilleartigen Geruchsnoten aus. Des weiteren sind blumige Noten, insbesondere Richtung Nelke wahrnehmbar.

Die bevorzugten Verbindungen sind das Methyl- und das Isopropylderivat (falls $R^2$ = H) und das Isopropylderivat (falls $R^2$ = Propionyl ).

Die folgende Tabelle I beschreibt die Gerüche der phenolischen Aether der Formel I.

**Tabelle I**

| $R^1$ | Geruch |
|---|---|
| $CH_3$ | nach Isoeugenol, Vanille, rauchig-phenolisch |
| $C_2H_5$ | würzig, nach Orangen, phenolisch |
| n-$C_3H_7$ | modrig, nach Methyleugenol, nach Vanille |
| iso-$C_3H_7$* | würzig, nach Isoeugenol, rauchig |

*Neue Verbindung, die per se als auch ihre Herstellung ebenfalls Teil der vorliegenden Erfindung ist.

Die Verbindungen, worin $R^1$ Isopropyl oder Methyl ist, sind, wie oben gesagt, für den vorliegenden Zweck besonders bevorzugt. Beide riechen intensiv und sind von sehr guter Haftfestigkeit, wobei bezüglich dieser Eigenschaften das Isopropyl-Analoge bevorzugt ist, weil es noch intensiver riecht.

Die Tabelle II beschreibt die Gerüche von Alkanoylderivaten I.

**Tabelle II**

| $R^1$ | $R^2$ | Geruch |
|---|---|---|
| $CH_3$ | $CH_3CO$ | nach Vanillin, natürlich, ein wenig phenolisch, an Isoeugenol erinnernd |
| $C_2H_5$ | $CH_3CO$ | würzig, pudrige Note |
| 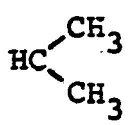 | $CH_3CO$ | würzig, nach Vanillebohnen, nach Nelken |
| $CH_3$ | $CH_3CH_2CO$ | blumig, würzig, butteriger Nebengeruch, nach Vanillin, |
| 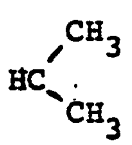 | $CH_3CH_2CO$ | würzig, vanilleartig, ätherisch |

Die Alkanoylderivate I sind neu, sie sind per se als auch ihre Herstellung ebenfalls Teil der vorliegenden Erfindung.

Die Verbindungen der Formel I ($R^2$ =H)eignen sich aufgrund ihrer Geruchsnoten insbesondere als Ersatz für Isoeugenol, welches seit einiger Zeit wegen seiner hautirritierenden Eigenschaften immer weniger verwendet wird. Daneben eignen sie sich zur Modifizierung einer ausgesprochenen breiten Palette von bekannten, z. B.

a) blumigen Kompositionen,z. B. Rose, Lilie, Narzisse, in denen z. B. die warmen, würzigen Noten verstärkt zum Ausdruck kommen sollen (z. B. für Herren-Cologne),

b) des weiteren aber auch von fruchtigen, von Lavendel und von Chypre-Kompositionen (Extrait-Typen, Kompositionen der femininen Richtung), von

c) Tabak- und Holz- und Fougére-Kompositionen, (Extrait-Typen der masculinen Richtung) und von

d) Kompositionen mit grünen Noten, wo insbesondere eine erwünschte Abrundung und harmonisierende Effekte erzielt werden.

Als Riechstoffe eignen sich die Verbindungen I auf Grund ihrer oben beschriebenen, originellen Noten, insbesondere in Kombination mit einer Reihe von natürlichen und synthetischen Riechstoffen, wie z. B.

- **Naturprodukten**

wie Angelikawurzöl, Galbanumöl, Vetiveröl, Patchouliöl, Sandelholzöl, Mandarinöl, Muskatellersalbei, Ylang-Ylang-öl, Cedernöl, Fichtenöl, Lavendelöl, Bergamotteöl, Citronenöl, Orangenöl, Corianderöl, Eichenmoos, Castoreum, Ciste labdanum, Calmusöl, Geraniumöl, Jasmin absolue, Rosenöl, Cassis absolue, Narzissen absolue, Verveine absolue, Grapefruit-Extrakten, usw.

- **Aldehyden**

wie $C_{10}$-, $C_{11}$-, $C_{14}$-aldehyd, Hydroxycitronellal, Cyklamenaldehyd, Benzaldehyd, p-tert.-Butyl-α-methylhydrozimtaldehyd, Citral, Citronellal, 2,6-Dimethyl-5-hepten-1-al, Isovaleraldehyd, trans-2-Hexenal, trans-2-Octenal, n-Octanal, n-Nonanal, trans-2-cis-6-Nonadienal, 2,4-Decadienal, Methylnonyl-acetaldehyd, Cyclal C(l,3,Dimethyl-cyclohex-1-en 4(und-5)-carboxaldehyd), Lyral, usw.

- **Ketonen**

wie alpha-Jonon, beta-Jonon, Methyljonon, Allyljonon, Acetanisol, 4-(para-Hydroxyphenyl)-2-butanon, Campher, Menthon, Carvon, Pulegon, p-Methylacetophenon, Methylamylketon, usw.

- **Acetalen und Ketalen**

wie Phenylacetaldehyd-dimethylacetal, Phenylacetaldehyd-glycerinacetal, 2-Methyl-1,3-dioxolan-2-äthylacetat, Capronaldehyd-dimethylacetal, Acetal R (gemischtes Acetal von Acetaldehyd mit Phenyläthylalkohol und n-Propanol), usw.

- **Äthern**

wie Eugenolmethyläther, Methyl-1-methyl-cyclododecyl-äther, Anethol, Estragol, Rosantolène (Methyläthylsaligenin), usw.

- **Phenolkörpern**

wie Vanillin, Eugenol, Creosol, Chavicol, usw.

- **Alkoholen**

wie Butanol, n-Hexanol, cis-3-Hexenol, trans-2-cis-6-Nonadienol, cis-6-Nonenol, Linalool, Geraniol, Rhodinol, Nerol, Citronellol, Nerolidol, Farnesol, Benzylalkohol, Phenyläthylalkohol, Zimtalkohol, Terpineol, Patchone (4-tert.-Butylcyclohexanol), usw.

- **Estern**

wie Aethylformiat, Aethylacetat, Isoamylacetat, t-Butylcyclohexylacetat, Myraldylacetat® (Givaudan), Benzylacetat, Styrallylacetat, Äthyl-α-methyl-phenylglycidat, Maltylisobutyrat, Dimethylbenzylcarbinylacetat und butyrat, Linalylacetat, Isobutylacetat, n-Amylbutyrat, n-Amylvalerianat, Äthylpalmitat, Cinnamylformiat, Terpenylacetat, Geranylacetat, Hexylsalicylat, Linalylanthranilat, Amylsalicylat, Methyldihydrojasmonat, Benzylsalicylat.

- **Lactone**

wie γ-Undecalacton, γ-Decalacton, γ-Nonalacton, δ-Decalacton, δ-Octalacton, Cumarin, usw.

- **Säuren**

wie Geranylsäure, Citronellylsäure, Zimtsäure, Phenylessigsäure, usw.

- **schwefelhaltigen Verbindungen**

wie p-Menthan-8-thiol-3-on, Dimethylsulfid und anderen Sulfiden und Disulfiden, usw.

- **stickstoffhaltigen Verbindungen**

wie Methylanthranilat, Indol, Isobutylchinolin, verschiedenen Pyrazinen, 5-Methyl-heptan-3-on-oxim, Nitromoschus, usw.

- **verschiedenen weiteren in der Parfümerie oft benutzten Komponenten**

wie Keton-Moschus, macrocyclische Moschuskörper wie Musk 174® (12-Oxahexadecanolid), Sandela ( Isocamphylcyclohexanol), polycyclische Moschuskörper wie Fixolid, Galaxolid.

Die Verbindungen der Formel I lassen sich in weiten Grenzen einsetzen, die beispielsweise von 0,1 (Detergentien) - 50 % (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen 0,5 und 20 %. Die mit 1 hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Desodorantien, Detergentien, Raumparfums, etc.).

Die Verbindungen 1 können demgemäss bei der Herstellung von Kompositionen und - wie obige Zusammenstellung zeigtunter Verwendung einer breiten Palette bekannter Riechstoffe verwendet werden. Bei der Herstellung solcher Kompositionen können die oben aufgeführten bekannten Riechstoffe nach (dem Parfümeur bekannter) Art und Weise verwendet werden, wie z. B. aus W.A. Poucher, Perfumes, Cosmetics and Soaps 2, 7. Auflage, Chapman und Hall, London, 1974 hervorgehend.

Die Herstellung von Vanillyläthern wird beispielsweise von Adler und Hernestam in Acta Chemica Scandinavica 9 (1955) 319 - 334, insbesondere 331 beschrieben. Im Gegensatz zu diesem bekannten Verfahren, bei dem man - zwecks Herstellung von 4-Hydroxy-3-methoxybenzyläthyläther - während 3 Stunden eine äthanolische HCL-Lösung zu einer eisgekühlten äthanolischen Vanillylalkohol-Lösung tropft und das Gemisch 16 Stunden bei Raumtemperatur reagieren lässt, wurde im Laufe der vorliegenden Arbeiten eine wesentlich einfachere Methode zur Herstellung der Äther I aus dem entsprechenden Benzylalkohol entwickelt: Man löst den Äthylvanillylalkohol im geeigneten $C_{1-3}$-Alkanol auf, gibt Natriumhydrogensulfat zu, erwärmt wenige Minuten, zweckmässigerweise auf Temperaturen von $\leqslant 50°C$, lässt abkühlen und arbeitet auf, indem man das Natriumhydrogensulfat neutralisiert und den $C_{1-3}$-Alkanol abdampft.

Dabei beträgt das Verhältnis 3-Äthylvanillylalkohol: $C_{1-3}$-Alkanol zweckmässigerweise mindestens 1:1, doch kann der $C_{1-3}$ -Alkanol auch im Ueberschuss vorliegen.

Das Natriumhydrogensulfat wird zweckmässigerweise in etwa derselben molaren Menge wie der Vanillylalkohol zugegeben, doch kann man auch schon mit einem Unterschuss, beispielsweise einem 20%igen Unterschuss, das erstrebte Ziel erreichen.

Die Alkanoylderivate

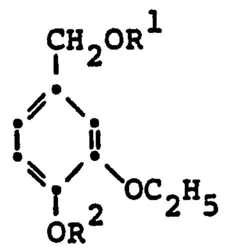

I''

worin $R^1$ $C_{1-3}$ -Alkyl und $R^2$ $C_{1-4}$-Alkanoyl bedeutet,
werden erhalten, indem man eine Verbindung der Formel

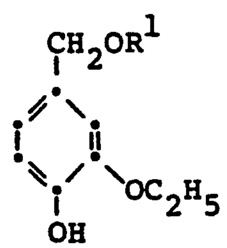

I'

worin $R_1$ obige Bedeutung hat, verestert.

Die Veresterung der Phenole . I' wird in an sich bekannter Weise unter Verwendung der üblichen Acylierungsmittel, also z. B. Acylhalogeniden oder Säureanhydriden durchgeführt. Bevorzugt ist die Arbeitsweise unter Verwendung der Säureanhydride. Man arbeitet zweckmässigerweise in Anwesenheit von Mineralsäuren, z. B. $H_3PO_4$ oder in Anwesenheit des Alkalimetallsalzes der entsprechenden Carbonsäure.

Gegenüber den aus Kokai No. 82308/82 und No. 9729/82 (Takasago) als Geschmackstoffe mit kühlender Wirkung, als Zusätze für scharfe Gewürze und als Aktivkomponenten für ziehende Pflaster bekanntgewordenen entsprechenden Methylderivaten der Formel

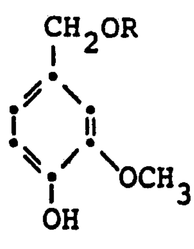

III

worin R $C_{1-6}$ -Alkyl darstellt, sind die vorliegenden phenolischen Verbindungen I insbesondere geruchlich wesentlich intensiver, im Durchschnitt und unter denselben Bedingungen verglichen, ca. 3 mal stärker, was sich insbesondere - aber nicht nur - in der Fond-(dry out)note auswirkt.

Die Alkanoylderivate sind, verglichen mit den phenolischen Derivaten,zwar geruchsschwächer, hingegen zeichnen sie sich insbesondere durch ausgezeichnete Haftfestigkeit aus.

**Beispiel 1**

100 g Äthylvanillylalkohol (4-Hydroxy-3-äthoxybenzyl-alkohol) werden in 200 ml Methanol gelöst. Man versetzt die Lösung mit 10 g Natriumhydrogensulfat und einer Messerspitze Hydrochinon. Man erwärmt zunächst 2 Minuten auf 50°C und lässt wieder auf Zimmertemperatur abkühlen. Man giesst nun die methanolische Lösung auf 85 ml einer gesättigten Natriumhydrogencarbonat-lösung. Der Methylalkohol wird im Vakuum abdestilliert, der Rückstand wird in Toluol aufgenommen, die organische Lösung mit Wasser neutral gewaschen und eingedampft. Der Rückstand (95,5 g) wird fraktioniert destilliert. Man erhält 61,1 g chemisch und olfaktisch reinen 4-Hydroxy-3-äthoxybenzylmethyläther; Sdp 77°C/0,04 mbar (0,03 mm Hg) $d_4^{20}$ = 1,1084; $n_D^{20}$ = 1,529.

Ausbeute : 56,3 %.

Auf analoge Weise werden, ebenfalls ausgehend aus Äthylvanillyl-alkohol, die folgenden Äther I ($R^2$ = H), Hergestellt:

| | | |
|---|---|---|
| $R^1$ = Äthyl | Sdp = 70°C0,09 mbar (0,07 mmHg,) | $d_4^{20}$ = 1,0756<br>$n_D^{20}$ = 1,521 |
| $R^1$ = n-Propyl | Sdp = 101°C/0,27mbar (0,2 mmHg,) | $d_4^{20}$ = 1,0528,<br>$n_D^{20}$ = 1,515 |
| $R^1$ = Isopropyl | Sdp = 98°/0,73 mbar (0,55 mmHg,) | $d_4^{20}$ = 1,0489<br>$n_D^{20}$ = 1,512 |

alle Derivate I ($R^2$ = H) sind ausgesprochen hitze- und lichtstabil.

**Beispiel 2**

In einen mit Rührer, Thermometer, Kühler und Tropftrichter versehenen Rundkolben gibt man 100 ml trockenes Toluol, 2,3 g Natriumpropionat und 31,5 g (0,15 Mol) 4-Hydroxy-3-äthoxybenzylisopropyläther. Innerhalb 30 Minuten werden unter Rühren bei Raumtemperatur 23 g (0,176 Mol) Propionanhydrid zugetropft. Nach der Zugabe wird das Reaktionsgemisch während 2 Stunden bei Rückflusstemperatur gehalten. Nach dem Abkühlen wird der Inhalt des Kolbens auf 100 g Eis gegossen, die organische Schicht abgetrennt, und dreimal mit 50 ml 10 %-iger Natriumcarbonatlösung gewaschen. Nach dem Trocknen über Natriumsulfat wird das Lösungsmittel eingedampft und der Rückstand (42 g) destilliert. Die fraktionierte Destillation ergibt 35 g (Ausbeute: 87,5 % der Theorie) chemisch reinen Propionsäureester, davon sind 19 g ohne weitere Behandlung olfaktisch reines Produkt; Sdp = 104°C/0,13 mbar (0,1 mmHg;) $d_4^{20}$ = 1,0349; $n_D^{20}$ = 1,4877. Der Rest kann durch zusätzliche Rektifizierung in olfaktisch reines Produkt übergeführt werden.

Auf analoge Weise werden die folgenden Alkanoylderivate aus den Aethern I ($R^2$ = H) hergestellt:

| | | |
|---|---|---|
| $R^1$ = Methyl | $R^2$ = Acetyl | Sdp = 91°C 0,23 mbar (0,17 mmHg)<br>$d_4^{20}$ = 1,0997,<br>$n_D^{20}$ = 1,5022 |
| $R^1$ Äthyl | $R^2$ = Acetyl | Sdp = 96°C/0,21 mbar (0,16 mmHgf),<br>$d_4^{20}$ = 1,0715<br>$n_D^{20}$ = 1,4958 |
| $R^1$ = Isopropyl | $R^2$ = Acetyl | Sdp = 98°C/0,20 mbar (0,15 mmHg),<br>$d_4^{20}$ = 1,0509<br>$n_D^{20}$ = 1,4911 |
| $R^1$ = Methyl | $R^2$ = Propionyl | Sdp = 89°C/0,07 mbar (0,05 mmHg),<br>$d_4^{20}$ = 1,0783<br>$n_D^{20}$ = 1,4976 |

**Beispiel 3**

A. Blumige Base Richtung Nelke

| | Gewichtsteile |
|---|---|
| Dipropylenglykol | 100 |
| Terpineol | 260 |
| Hydroxycitronellal | 220 |
| Zimtalkohol-substitut | 120 |
| Phenyläthylalkohol | 100 |
| Cinnamylformiat | 20 |
| Linalool | 15 |
| Terpenylacetat | 10 |
| Keton-Moschus | 10 |
| Geranylacetat | 10 |
| Jasmin synthetisch | 10 |
| Eugenol | 5 |
| Indol 10 % in Dipropylenglykol(DPG) | 5 |
| $C_{10}$-Aldehyd 10 % in DPG | 5 |
| p-Methylacetophenon | 5 |
| Undecalacton | 5 |
| | 900 |

Durch Zugabe von 100 Teilen des Methylderivats I ($R^2$ = H) gewinnt die Base einen sehr angenehmen pudrig-blumigen Charakter mit leichter Grünnote Richtung Nelke. Ein analoger Effekt kann durch Zugabe von 100 Teilen Isoeugenol erzielt werden. Zugabe von 100 Teilen des Isopropylderivats I ($R^2$ = H) erbringt in der Base eine angenehme Süsse und Volumen; durch die Zugabe wird der Komplex Jasmin-Flieder in eine pudrigwürzige Note eingekleidet, mit anderen Worten: Es entsteht die typische Nelkennote. Gibt man andererseits zu der obigen Base allgemein blumiger Richtung 100 Teile 4-Hydroxy-3-methoxybenzylmethyläther oder 100 Teile 4-Hydroxy-3-methoxybenzylisopropyläther, wird die Base geruchlich aus dem Gleichgewicht gebracht. Die Geruchsnote erhält einen harten Charakter; das blumige Element wird völlig verdrängt.

B. Nelken-Base

| | Gewichtsteile |
|---|---|
| Eugenol | 250 |
| Phenyläthylalkohol | 100 |
| Dipropylenglykol | 70 |
| Terpineol | 60 |
| Ylang-Ylang-öl | 50 |
| Benzylsalicylat | 50 |
| α-Jonon | 30 |
| Hydroxycitronellal | 30 |
| Amylsalicylat | 20 |
| Rhodinol | 80 |
| Phenylacetaldehyd | 10 |
| | 750 |

Durch Zugabe von 250 Teilen des Methylderivats I ($R^2$ = H) zu der obigen blumigen Base wird diese Base sehr angenehm eingekleidet. Es entwickelt sich ein sehr weicher pudriger Nelkengeruch. Auch der Zusatz von 250 Teilen des Isopropylderivats I ($R^2$ = H) wirkt sich im Entstehen einer sehr angenehmen pudrigen und abrundenden Geruchsnote Richtung Nelke aus. Auch durch Zusetzen von 250 Teilen Isoeugenol erhält die blumige Base ihren typischen Nelkencharakter mit der etwas herben Grünnote. Setzt man andererseits 250 Teile 4-Hydroxy-3-methoxybenzylmethyläther bzw. 4-Hydroxy-3-methoxybenzylisopropyläther zu, so wird die Base geruchlich aus dem Gleichgewicht gebracht. Sie wirkt nun flach und das Eugenol dominiert, so dass ein terpeniger Eindruck entsteht.

**Beispiel 4**

In diesem Beispiel wird gezeigt, wie - in jeder Hinsicht konventionelle - Basen durch Zusatz von Verbindungen I auf unerwartete und erwünschte Weise veredelt werden können. Die Zusätze betragen jeweils 10 Teile. Eine Veredlung mittels Isoeugenol andererseits konnte hier in keinem Fall beobachtet werden.

Bei den gegenständlichen Zusätzen handelt es sich um:

4-Hydroxy-3-äthoxybenzylmethyläther (A)
4-Hydroxy-3-äthoxybenzylisopropyläther (B)
4-Propionyloxy-3-äthoxybenzylisopropyläther (C)
4-Acetoxy-3-äthoxybenzylmethyläther (D)

A. Parfumerie-Base Richtung Rose

| | Gewichtsteile |
|---|---|
| Phenyläthylalkohol | 300 |
| Geraniol extra | 300 |
| Jasmin synthetisch | 240 |
| Citronellol | 100 |
| α-Jonon | 40 |
| $C_{10}$-Aldehyd 10 % in Dipropylenglykol | 5 |
| $C_{11}$-Aldehyd 10 % in Dipropylenglykol | 5 |
| | 990 |

Durch Zusatz von 10 Teilen A entsteht eine sehr angenehm Pudrige und würzige Rose, die in Richtung helle Rose tendiert.

Ein Zusatz von 10 Teilen C bringt sehr viel Wärme und Fülle in die Base. Es entsteht eine sehr runde dunkle Rose.

Durch 10 Teile B wird die Base sehr pudrig, sehr süss und schwer.

B. Parfumerie-Base Richtung Narzisse

| | Gewichtsteile |
|---|---|
| Hydroxycitronellal | 400 |
| Phenyläthylalkohol | 400 |
| Aubépine (p-anisaldehyd, ex Anethol) | 50 |
| p-Cresyl-acetat 10 % DPG | 30 |
| Ylang-Ylang-öl | 20 |
| Dipropylenglykol | 90 |
| | 990 |

10 Teile A bringen die Base zwar aus der Richtung Narzisse in Richtung Flieder. Die Base wird aber viel wärmer und pudriger, der Aubépine-Charakter wird stark unterstrichen.

Mit 10 Teilen D wird die Narzissennote der Base wesentlich kräftiger und typischer.

Auch durch Zusatz von 10 Teilen C wird die Base blumiger, zudem weicher und sehr schön abgerundet.

C. Parfumerie-Base Richtung Chypre

| | Gewichtsteile |
|---|---|
| Raldein (Gemisch von Methyl- jononen) | 200 |
| Keton-Moschus | 100 |
| Phenyläthylalkohol | 80 |
| Linalylacetat | 70 |
| Baummoos absolut | 50 |
| Vetivenylacetat | 50 |
| Methyldihydrojasmonat | 50 |
| α-Hexylzimtaldehyd | 50 |
| Patchouliöl | 30 |
| Citronellöl ex Geraniumöl | 30 |
| Eugenol | 30 |
| Sandela Givaudan (Isocamphyl-cyclohexanol) | 30 |
| Zedernholzöl | 30 |
| Styrallylacetat | 20 |
| Galbanumöl | 10 |
| Neroliöl | 10 |
| Castoreumöl 10 % DPG | 10 |
| Isobutylchinolin 10 % DPG | 10 |
| Armoiseöl | 10 |
| $C_{11}$-Aldehyd 10 % DPG | 10 |
| Citral | 5 |
| Undecalacton | 5 |
| Cistusöl | 5 |
| Dipropylenglykol | 95 |
| | 990 |

Ein Zusatz von 10 Teilen A erbringt in der Chypre-Base eine sehr angenehm pudrige, warme Note, welche sehr abrundend wirkt.

Ein Zusatz von 10 Teilen B wirkt ebenfalls äusserst abrundend. Die Base wird viel weicher, der Komplex Neroli-Keton-Moschus wird harmonisch unterstrichen; sehr gut geeignet für weibliche Linien.

Der Zusatz von 10 Teilen C unterstreicht hingegen die holzige, etwas würzige Note des Komplexes Vetiver-Patchouli-Zedernholz; sehr gut für maskuline Linien geeignet.

D. Parfumerie-Base Richtung Fougère

| | Gewichtsteile |
|---|---|
| Baummoos absolut | 60 |
| Lavendelöl französisch | 200 |
| Linalylacetat | 150 |
| Cumarin | 50 |
| Patchouliöl | 30 |
| Citronellöl ex Geraniumöl ("Rhodinol") | 30 |
| Methyldihydrojasmonat | 30 |
| Ketonmoschus | 30 |
| Vetivenylacetat | 30 |
| Geranium Bourbon synthetisch | 30 |
| Amylsalicylat | 20 |
| Sandela Givaudan | 20 |
| Linalool | 20 |
| Benzylacetat | 15 |
| Ylang-Ylang-öl | 15 |
| Eugenol | 15 |
| Thymianöl | 5 |
| Dipropylenglykol | 240 |
| | 990 |

Gibt man zu obiger Fougère-Base 10 Teile A, so wird der würzige Cumarin-Aspekt sehr gut unterstrichen.

Gibt man jedoch D zu, so wird die Base viel holziger. Nun wird der Komplex Patchouli-Sandela sehr vorteilhaft abgerundet.

Gibt man jedoch C zu der Base, wirkt diese sofort sehr viel wärmer. Die etwas harte Salicylat-Lavendel-Note wirkt nun angenehm abgerundet, indem sie sich mit der blumigen Komponente des Rhodinols / Methyldihydrojasmonates verbindet.

E. Frisch-grüne Base

| | Gewichtsteile |
|---|---|
| Linalylacetat | 200 |
| Linalool synthetisch | 200 |
| Lavandin ess. France | 200 |
| Landenol (Gemisch von Tetrahydro- linalool, Tetrahydromyrcenol, 3,7-Dimethyl-4-octen-3-ol, 2,6-Dimethyl-3-octen-2-ol) | 150 |
| Rosmarinöl | 50 |
| Pfefferminzöl Brasilien | 20 |
| Salbeiöl französisch | 20 |
| Ambersage Givaudan (4,7-Dihydro-2- isopentyl-2-methyl-1,3-dioxepin) | 20 |
| Eucalyptol | 20 |
| Fichtennadelöl | 10 |
| Methylsalicylat 10 % DPG | 5 |
| Eugenol | 5 |
| Dione Givaudan (2-[3,3,5-Trimethyl-cyclohexylacetyl]-cyclopentanon) | 5 |
| Galbanumöl (natürlich) | 5 |
| Dipropylenglykol | 80 |
| | 990 |

Durch Zusatz von 10 Teilen B erhält obige Base einen sehr warmen, pudrig süssen Charakter, der etwas harte Effekt von Methylsalicylat und Galbanum wirkt nun sehr vorteilhaft eingekleidet.

Ein Zusatz von 10 Teilen C kleidet die Base sehr angenehm ein; der Gewinn an Volumen und Diffusion ermöglicht deren Verwendung für Kompositionen der Richtung feminine Linien.

**Patentansprüche**

1. Riechstoffkomposition, gekennzeichnet durch einen Gehalt an einer Verbindung der Formel

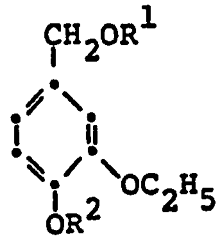

I

worin $R^1$ $C_{1-3}$ -Alkyl und $R^2$ Wasserstoff oder $C_{1-4}$-Alkanoyl bedeutet.

2. Riechstoffkomposition, gekennzeichnet durch einen Gehalt an einer Verbindung der Formel

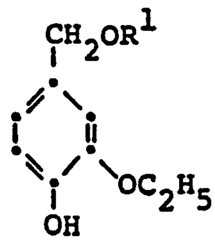

I'

worin $R^1$ einen $C_{1-3}$-Alkylrest darstellt.

3. Riechstoffkomposition gekennzeichnet durch einen Gehalt an 4-Hydroxy-3-äthoxybenzylmethyläther.

4. Riechstoffkomposition gekennzeichnet durch einen Gehalt an 4-Hydroxy-3-äthoxybenzylisopropyläther.

5. Riechstoffkomposition gekennzeichnet durch einen Gehalt an 4-Propionyloxy-3-äthoxybenzylisopropyläther.

6. Verwendung einer Verbindung der Formel

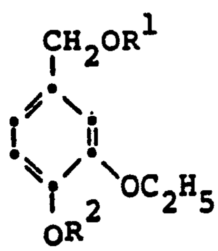

I

worin $R^1$ $C_{1-3}$-Alkyl und $R^2$ Wasserstoff oder $C_{1-4}$-Alkanoyl bedeutet, als Riechstoff.

7. Verwendung einer Verbindung der Formel

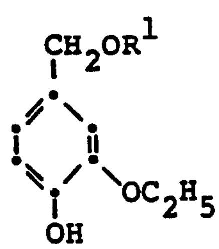

I'

worin $R^1$ einen $C_{1-3}$-Alkylrest darstellt, als Riechstoff.

8. Verwendung von 4-Hydroxy-3-äthoxybenzylmethyläther als Riechstoff.

9. Verwendung von 4-Hydroxy-3-äthoxybenzylisopropyl-äther als Riechstoff.

10. Verwendung von 4-Propionyloxy-3-äthoxybenzylisopropyl-äther als Riechstoff.

11. 4-Hydroxy-3-äthoxybenzylisopropyläther.

12. Verfahren zur Herstellung von 4-Hydroxy-3-äthoxy-benzylisopropyläther, dadurch gekennzeichnet, dass man 4-Hydroxy-3-äthoxybenzylalkohol mit Isopropanol umsetzt.

13. Verbindungen der Formel

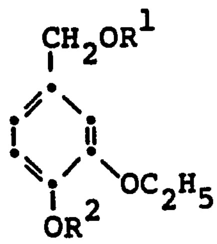

I''

worin
$R^1$ $C_{1-3}$-Alkyl und $R^2$ $C_{1-4}$-Alkanoyl bedeutet.

14. 4-Propionyloxy-3-äthoxybenzylisopropyläther.
15. 4-Propionyloxy-3-äthoxybenzylmethyläther.
16. 4-Acetyloxy-3-äthoxybenzylmethyläther.
17. 4-Acetyloxy-3-äthoxybenzyläthyläther.
18. 4-Acetyloxy-3-äthoxybenzylisopropyläther.
19. Verfahren zur Herstellung von Verbindungen der Formel

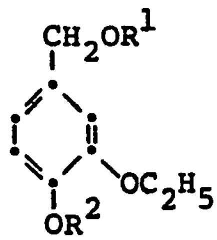

I''

worin R einen $C_{1-3}$-Alkylrest darstellt und $R^2$ $C_{1-4}$-Alkanoyl ist, dadurch gekennzeichnet, dass man eine Verbindung der Formel

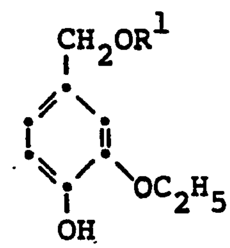

I'

worin $R_1$ obige Bedeutung hat, verestert.

## Claims

1. An odorant composition characterized by a content of a compound of the formula

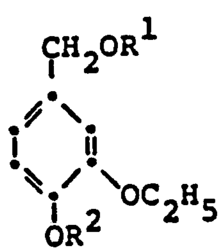

I

wherein $R^1$ signifies $c_{1-3}$-alkyl and $R^2$ signifies hydrogen or $C_{1-4}$-alkanoyl.

2. An odorant composition characterized by a content of a compound of the formula

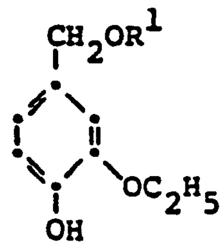

I'

wherein $R^1$ represents a $C_{1-3}$-alkyl residue.

3. An odorant composition characterized by a content of 4-hydroxy-3-ethoxybenzyl methyl ether.
4. An odorant composition characterized by a content of 4-hydroxy-3-ethoxybenzyl isopropyl ether.
5. An odorant composition characterized by a content of 4-propionyloxy-3-ethoxybenzyl isopropyl ether.
6. The use of a compound of the formula

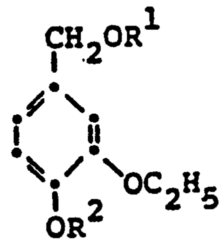

I"

wherein $R^1$ signifies $C_{1-3}$-alkyl and $R^2$ signifies hydrogen or $C_{1-4}$-alkanoyl, as an odorant.

7. The use of a compound of the formula

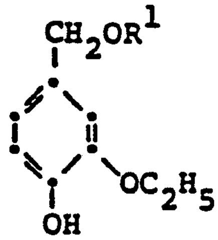

I'

wherein $R^1$ represents a $C_{1-3}$-alkyl residue, as an odorant.

8. The use of 4-hydroxy-3-ethoxybenzyl methyl ether as an odorant.
9. The use of 4-hydroxy-3-ethoxybenzyl isopropyl ether as an odorant.
10. The use of 4-propionyloxy-3-ethoxybenzyl isopropyl ether as an odorant.
11. 4-Hydroxy-3-ethoxybenzyl isopropyl ether.
12. A process for the manufacture of 4-hydroxy-3-ethoxybenzyl isopropyl ether, characterized by reacting 4-hydroxy-3-ethoxybenzyl alcohol with isopropanol.
13. Compounds of the formula

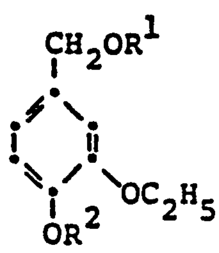

I"

wherein $R^1$ signifies $C_{1-3}$-alkyl and $R^2$ signifies $C_{1-4}$-alkanoyl.

14. 4-Propionyloxy-3-ethoxybenzyl isopropyl ether.
15. 4-Propionyloxy-3-ethoxybenzyl methyl ether.
16. 4-Acetyloxy-3-ethoxybenzyl methyl ether.
17. 4-Acetyloxy-3-ethoxybenzyl ethyl ether.
18. 4 -Acetyloxy-3-ethoxybenzyl isopropyl ether.
19. A process of the manufacture of compounds of the formula

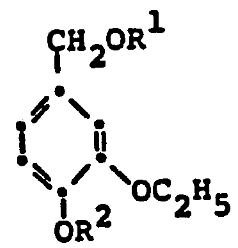

I

wherein R represents a $C_{1-3}$-alkyl residue and $R^2$ is $C_{1-4}$-alkanoyl, characterized by esterifying a compound of the formula

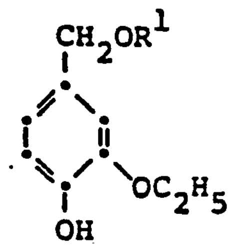

I'

wherein $R^1$ has the above significance.

**Revendications**

1. Composition odorante, caractérisée en ce qu'elle contient un composé de formule

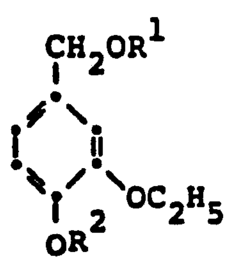

I

où R1 représente un alcoyle en C1 à C3 et R2 un hydrogène ou un alcancyle en C1 à C4.

2. Composition odorante caractérisée en ce qu'elle contient un composé de formule

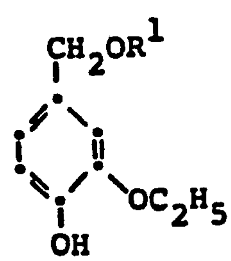

I'

où R1 représente un radical alcoyle en C1 à C3.

3. Composition odorante caractérisée en ce qu'elle contient du 4-hydroxy-3-éthoxybenzylméthyléther.

4. Composition odorante caractérisée en ce qu'elle contient du 4-hydroxy-3-éthoxybenzylisopropyléther.

5. Composition odorante caractérisée en ce qu'elle contient du 4-propio-nyloxy-3-éthoxybenzylisopropyléther.

6. Application d'un composé de formule

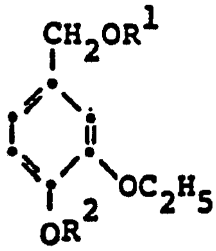

I

où R1 représente un alcoyle en C1 à C3 et R2 un hydrcgène ou un alcancyle en C1 à C4, comme odorant.

7. Application d'un composé de formule

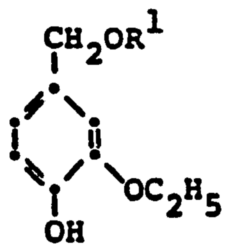

I'

où R1 représente un radical alcoyle en C1 à C3, comme odorant.

8. Application de 4-hydroxy-3-éthoxybenzylméthyléther comme odorant.

9. Application de 4-hydroxy-3-éthoxyhenzylisopropyléther comme odorant.

10. Application de 4-propionyloxy-3-éthoxybenzylisopropyléther comme odorant.

11. 4-hydroxy-3-éthoxybenzylsopropyléther.

12. Procédé de préparation de 4-hydroxy-3-éthoxybenzylisopropyléther, caractérisé en te qu'on fait réagir du 4-hydroxy-3-éthoxybenzylalcool avec de l'isopropanol.

13. Composés de formule

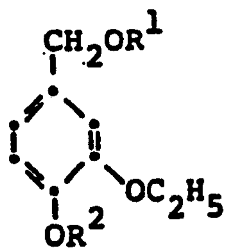

I''

où R1 représente un alcoyle en C1 à C3 et R2 un alcanoyle en C1 à C4.

14. 4-propionyloxy-3-éthoxybenzylisopropyléther.

15. 4-propionyloxy-3-éthoxybenzylméthyléther.

16. 4-acétyloxy-3-éthoxyhenzylméthyléther.

17. 4-acétyloxy-3-éthoxybenzyléthyléther.

18. 4-acétyloxy-3-éthoxyhenzylsopropyléther.

19. Procédé de préparation de composés de formule

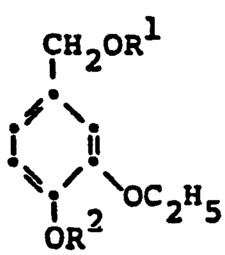

I''

où R représente un radical alcoyle en C1 à C3 et R2 est un alcanoyle en C1 à C4, caractérisé en ce qu'on estérifie un composé de formule

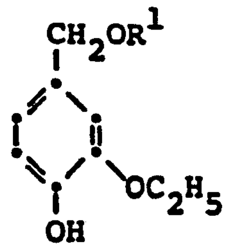

I'

où R1 a la signification ci-dessus.